# EUROPÄISCHE PATENTANMELDUNG

(11) **EP 2 399 983 A1**
(43) Veröffentlichungstag der Anmeldung: **28.12.2011**
(21) Anmeldenummer: 10450106.9
(22) Anmeldetag: 23.06.2010
(51) Int. Cl.: C12M 1/107

(54) **Erdbeckenfermenter**

(71) Anmelder: Sattler AG, 8041 Graz (AT)
(72) Erfinder: Wiedau, Helmut, 48161 Münster (DE)
(74) Vertreter: Ellmeyer, Wolfgang

(57) **Zusammenfassung**

Erdbeckenfermenter (1) mit einem abgesenkten Beckenboden (3), der mit einer Dichtungsschicht (31) abgedeckt ist und ein Erdbecken (7) ausbildet, und an dessen Beckenkrone (8) zumindest eine gasdichte Abdeckung (1; 33, 34) festgelegt ist, wobei Zu- und Ableitungen vorgesehen sind, wobei innerhalb der gasdichten Abdeckung (1; 33, 34) im Bereich der Beckenkrone (8) zumindest teilweise entlang des Umfangs der Beckenkrone (8) zumindest ein Rohr (19) angeordnet ist, das entlang seiner Längserstreckung Durchbrechungen oder Öffnungen in der Rohrwandung aufweist, durch die hindurch Gas in das Innere des Rohres eintreten oder aus dem Inneren des Rohres austreten kann.

## Beschreibung

Die Erfindung betrifft einen Erdbeckenfermenter mit einem abgesenkten Beckenboden, der mit einer Dichtungsschicht abgedeckt ist und ein Erdbecken ausbildet, und an dessen Beckenkrone zumindest eine gasdichte Abdeckung festgelegt ist, wobei Zu- und Ableitungen vorgesehen sind.

Die in letzter Zeit an Erdbeckenfermentern vorgenommenen Verbesserungen ermöglichen nunmehr eine durchdringungsfreie Abdeckung des Beckenbodens, indem die Zu- und Abführleitungen und diverse mechanische Antriebe durch die gasdichten Membran hindurchgeführt werden. Weiters ist es gelungen, den konstruktiven Aufwand dadurch zu verringern, dass nunmehr keine Stützgurte für die Gasmembran benötigt werden, sondern es wird die Gasmembran bis auf den Beckenboden niedersinken gelassen Dadurch kann es aber zu einer diskontinuierlichen Gaszu- oder abführung kommen, da die bis auf den Boden absinkende Gasmembran zur Ausbildung von lokalen Gaseinschlüssen führen kann, welche Gasvolumina ausbilden, zu denen keine Gaskommunikation in Richtung zur Auslassöffnung des Erdbeckenfermenters hin besteht. In umgekehrter Richtung kann auch der Gaseintritt ungleichmäßig verteilt sein, weil das einströmende Gas nicht in alle von der Gasmembran abgedeckten Volumsteile vordringen kann. Durch das Vorhandensein einer Gaseintritts- oder Gasaustrittsöffnung an nur einer Stelle des Erdbeckenfermenters, insbesondere wenn diese in der gasdichten Abdeckung vorgesehen ist, wird das beschriebene diskontinuierliche Verhalten noch verstärkt, da die Gaskommunikation mit verschiedenen Bereichen des Erdbeckenfermenters durch die absinkende Membran sehr leicht gestört oder unterbrochen werden kann.

Aufgabe der Erfindung ist es daher, einen Erdbeckenfermenter der eingangs genannten Art anzugeben, bei dem die Gaszu- und Abführung auch bei diskontinuierlichem Verhalten der Gasmembran kontrolliert erfolgt und das Ausbilden von lokalen Gaseinschlüssen durch die herabsinkende Gasmembran unterbunden wird.

Weitere Aufgabe der Erfindung ist die Sicherstellung der Gaskommunikation von nur einer Gaszu- oder abfuhröffnung aus.

Erfindungsgemäß wird dies dadurch erreicht, dass innerhalb der gasdichten Abdeckung im Bereich der Beckenkrone zumindest teilweise entlang des Umfangs der Beckenkrone zumindest ein Rohr angeordnet ist, das entlang seiner Längserstreckung Durchbrechungen oder Öffnungen in der Rohrwandung aufweist, durch die hindurch Gas in das Innere des Rohres eintreten oder aus dem Inneren des Rohres austreten kann.

Die erfindungsgemäße Anbringung des Rohres ermöglicht einerseits eine Zu- und Ableitung eines Gases entlang des gesamten Beckenumfanges oder Teilbereichen davon und somit die Herstellung einer Kommunikation mit allen Umfangsbereichen und andererseits wird durch das Anordnen des Rohres auf der Höhe der Beckenkrone vermieden, dass es zu einem Verlegen der Durchbrechungen oder Öffnungen im Rohr durch Substratbestandteile kommen kann. Durch die Ein- und Austrittsmöglichkeit entlang des Beckenumfanges werden lokale Gasansammlungen verhindert und es kann der gesamte Gasinhalt des Erdbeckenfermenters austreten oder Gas im gesamten Beckenvolumen eintreten. Weiters kann das im Rohr gesammelte Gas einer oder mehreren Austrittsöffnungen zugeführt werden. In umgekehrter Richtung gelangt ein dem Rohr über eine Eintrittsöffnung strömendes Gas über das erfindungsgemäß angeordnete Rohr in alle Umfangsbereiche des Erdbeckens.

Die Anbindung des zumindest einen Rohres an eine Gaszufuhr oder -abfuhröffnung kann gemäß einem Ausführungsbeispiel dadurch erfolgen, dass das zumindest eine Rohr durch eine offene Ringrohrleitung ausgebildet ist, deren offene Enden im Bereich einer Gaseintritts- oder einer Gasaustrittsöffnung angeordnet sind. Auf diese Weise ist die Gaskommunikation zwischen der Gaseintritts- oder der Gasaustrittsöffnung und den Bereichen entlang der Beckenkrone sichergestellt und die Ausbildung von Gaseinschlüssen kann auf einfache Weise vermieden werden.

Alternativ dazu könnte aber auch die Verbindung zwischen Rohr und einer Austritts-oder Eintrittsöffnung über eine dafür vorgesehene Verbindungsleitung geschehen.

Der seitliche Eintritt oder Austritt von Gas entlang der Rohrlänge kann dadurch erfolgen, dass die Durchbrechungen in der Rohrwandung durch eine Vielzahl von Längsschlitzen gebildet sind, die entlang der Längerstreckung des Rohres verteilt sind, wodurch das Gas ohne großen Strömungswiderstand ein- und austreten kann.

Eine sichere Art der Befestigung des Rohres innerhalb des Erdbeckenfermenters kann erfolgen, indem in weiterer Ausbildung der Erfindung in der Beckenkrone ein Betonringfundament eingelassen ist, und dass das Rohr auf dem Betonringfundament angeordnet und gegenüber diesem -oder mit der darauf befindlichen Dichtungsbahn durch Schweißlaschen- fixiert ist.

Gemäß einer weiteren Ausführungsform der Erfindung kann die gasdichte Abdeckung durch zumindest eine gasdichte Membran gebildet sein, wobei bevorzugt die zumindest eine gasdichte Membran durch eine Innenmembran und eine Außenmembran gebildet ist. Zwischen der Innenmembran und der Außenmembran wird durch eine Stützgasatmosphäre dafür gesorgt, dass die Außenmembran eine stets gleich bleibende Form beibehält, während die Innenmembran je nach Füllgrad sich senken und heben kann.

Eine wesentliche Schwachstelle bei Erdbeckenfermentern ist die Anbringung der gasdichten Abdeckung am Beckenrand, um eine dauerhafte Abdichtung zu erzielen.

Gemäß einer weiteren Ausführungsform der Erfindung kann deshalb die gasdichte Abdeckung im Randbereich durch eine Klemmschiene auf der Beckenkrone gasdicht geklemmt sein, wobei die Klemmschiene durch eine sich entlang des gesamten Umfangs der Beckenkrone erstreckende Klemmschiene gebildet ist.

Die Klemmschiene ist dabei lösbar angebracht, sodass diese jederzeit für Revisionsarbeiten im Inneren des erfindungsgemäßen Erdbeckenfermenters materialverlustfrei und ohne großen Aufwand geöffnet und wieder geschlossen werden kann.

Die Klemmschiene kann aus mehreren Einzelschienen zusammengesetzt sein.

Eine mechanisch besonders robuste Variante der Klemmschiene ist im Querschnitt gesehen durch ein Winkelprofil gebildet.

Die Fixierung der Klemmschiene kann durch im Betonringfundament verankerte Dübelverbindungen geschehen, die leicht lösbar sind, um die Klemmung aufzuheben und die Gasmembran(en) zu entfernen.

Die übliche Anbringung von Gurten zum Auflegen der absinkenden Gasmembran ist konstruktiv relativ aufwendig, da eine entsprechende statische Absicherung zu erfolgen hat. Es kann aber auf diese Gurtauflage verzichtet werden, indem die zumindest eine gasdichte Membran so konfektioniert ist, dass sie im entleerten Zustand des Gasspeichers zumindest teilweise auf dem Speicherboden flächig aufliegt.

Dies kann bevorzugt dadurch erfolgen, dass zumindest entlang einem Querschnitt durch den Gasspeicher die Bogenlänge b der zumindest einen Membran im aufgespannten Zustand größer oder gleich der zwischen gegenüberliegenden Einspannpunkten der zumindest einen gasdichten Membran gemessenen Länge der Durchlauflinie d entlang dem Speicherboden ist. Auf diese Weise kann das gesamte Flächengewicht der Gasmembran vom Untergrund aufgenommen werden.

Ein Problem bei Erdbeckenfermentern stellt die durch Witterungseinflüsse hervorgerufene Bodenerosion dar, die sich durch Abspülen der Außenböschungen und/oder Unterspülen von tragenden Bauteilen negativ auf die Bausubstanz auswirken kann. Um dies zu verhindern, kann gemäß einer Ausführungsform der Erfindung vorgesehen sein, dass außerhalb der gasdichten Abdeckung eine Vertiefung in der Beckenkrone vorgesehen ist, die sich entlang des Umfanges der Beckenkrone erstreckt, wobei in der Vertiefung ein Drainrohr zur Abführung des Regenwassers angeordnet ist.

Eine Verbesserung der Drainagewirkung kann dadurch erreicht werden, dass die Vertiefung mit Dichtungsbahnen ausgekleidet und mit Kies gefüllt ist.

In weiterer Ausbildung der Erfindung kann der abgesenkte Beckenboden Böschungsflächen aufweisen, auf welchen Heizungsrohre verlegt sind, die mit Abstandshalter montiert sein können, um eine Beschädigung der Beckenauskleidung zu vermeiden. Das Verlegen der Heizungsrohre auf den Böschungsflächen kann über eine sehr große Fläche erfolgen, wodurch ein lokales Überheizen des Substrats vermieden wird und zugleich sehr große Beckenbereiche gleichmäßig erwärmt werden können.

Die Erfindung wird nachfolgend anhand des in den Zeichnungen dargestellten Ausführungsbeispiels eingehend erläutert. Es zeigt dabei
Fig.1 eine Draufsicht auf eine Ausführungsform eines erfindungsgemäßen Erdbeckenfermenters;
Fig.2 einen Schnitt durch den Erdbeckenfermenter gemäß Fig.1;
Fig.3 einen teilweisen Schnitt durch eine weitere Ausführungsform des erfindungsgemäßen Erdbeckenfermenters;
Fig.4 eine teilweise dreidimensionale Ansicht des Innenraums des erfindungsgemäßen Erdbeckenfermenters gemäß Fig.3.

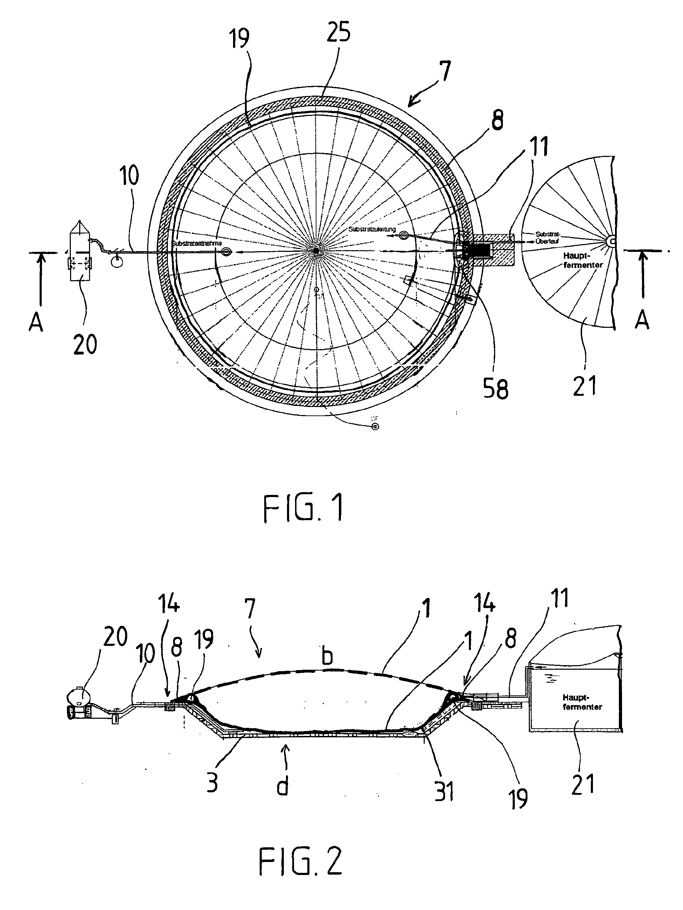

Fig.1 und 2 zeigen einen Erdbeckenfermenter mit einem abgesenkten Beckenboden 3, der mit einer Dichtungsschicht 31 abgedeckt ist und ein Erdbecken 7 mit kreisförmigem Grundriss ausbildet. Im gezeigten Ausführungsbeispiel verläuft der Beckenboden 3 mit schrägen Böschungen. Die Erfindung ist aber nicht auf eine bestimmte Bauform des Erdbeckenfermenters 1 beschränkt, weshalb auch senkrechte Böschungswände oder ein anderer Grundriss, z.B. ein rechteckförmiger Grundriss ausgeführt sein könnte.

Auf einem kreisförmig angelegten Betonringfundament 25, das in der Beckenkrone 8 des Erdbeckenfermenters 1 eingelassen ist, ist eine gasdichte Abdeckung festgelegt, die durch eine gasdichte Membran 1 gebildet ist, die mit ihrem Außenrand an der Beckenkrone 8 gasdicht geklemmt ist. Auch hinsichtlich der gasdichten Abdeckung sind alle dem Fachmann bekannten Variationen anwendbar, so ist z.B. die Anzahl und Form der gasdichten Membranen frei wählbar.

In Fig. 1 sind eine Substratzuleitung 11 aus einem Hauptfermenter 21 und eine zu einem Tankwagen 20 führende Substratableitung 10 gezeigt, die durch die Innen-und die Außenmembran 34, 33 gasdicht hindurchgeführt sind und über welche Substrat in den Erdbeckenfermenter eingeleitet bzw. abgeleitet werden kann.

Weitere nicht dargestellte Zu- und Ableitungen sind Gaszu- und ableitungen sowie gegebenenfalls Heizmedium Zu- und Ableitungen oder diverse andere elektrische Leitungen für Heiz- oder Messzwecke.

Es können z.B. Heizungsrohre auf Böschungsflächen des Beckenbodens 3 verlegt sein, welche mit Abstandshalter montiert sind und der Erwärmung des im Erdbeckenfermenters befindlichen Substrats dienen, wodurch die Fermentation beschleunigt werden kann. Um z.B. eine Beschädigung der Auskleidungen der Böschungsflächen zu vermeiden, können die Heizungsrohre auf Kunststoff-Abstandshaltern oder Altreifenteilen befestigt werden. Eine durch Wärmeeinwirkung verursachte Längenänderung der Heizungsrohrleitungen wirkt sich daher nicht durch das Ausbilden von Rohrspannungen aus.

Die gasdichte Membran 1 ist so konfektioniert, dass sie im entleerten Zustand des Gasspeichers 7 zumindest teilweise auf dem Beckenboden 3 flächig aufliegt.

Dies ist dadurch realisiert, dass entlang einem Querschnitt durch den Gasspeicher 7 die Bogenlänge b der Membran 1 (strichliert gezeichnet) im aufgespannten Zustand größer oder gleich der zwischen gegenüberliegenden Einspannpunkten 14 der gasdichten Membran 1 gemessenen Länge der Durchlauflinie d (ausgezogen gezeichnet) entlang dem Beckenboden 3 ist.

Auf diese Weise wird kein Gurt zum Auflegen der gasdichten Membran 1 benötigt, weil diese bis auf den Boden 3 absinken kann. Es kann durch dieses Absinken dabei aber an bestimmten Punkten zu Gaseinschlüssen kommen, die ein vollständiges Entleeren des Erdbeckenfermenters verhindern würden.

Erfindungsgemäß ist daher innerhalb der gasdichten Abdeckung 1 im Bereich der Beckenkrone 8 entlang des Umfangs der Beckenkrone 8 zumindest ein Rohr 19 angeordnet, das entlang seiner Längserstreckung Durchbrechungen oder Öffnungen in der Rohrwandung aufweist, durch die hindurch Gas in das Innere des Rohres 19 eintreten oder aus dem Inneren des Rohres 19 austreten kann.

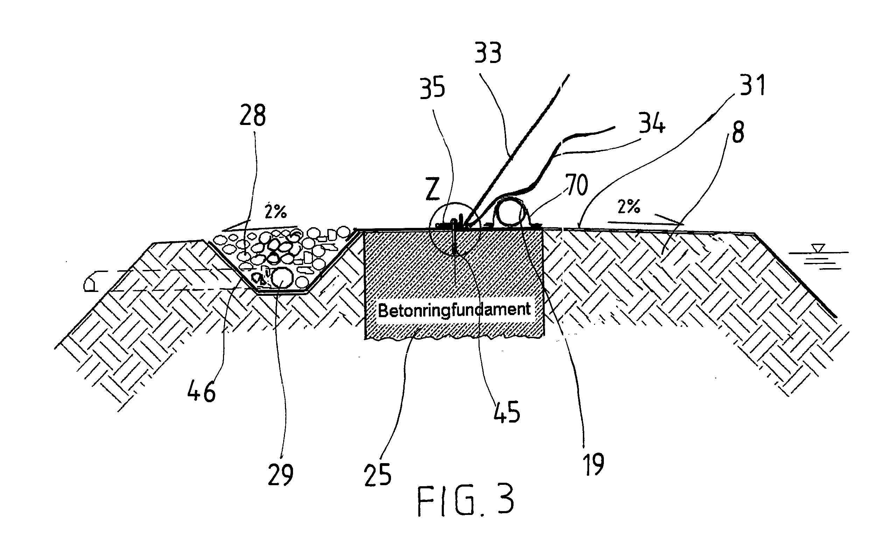

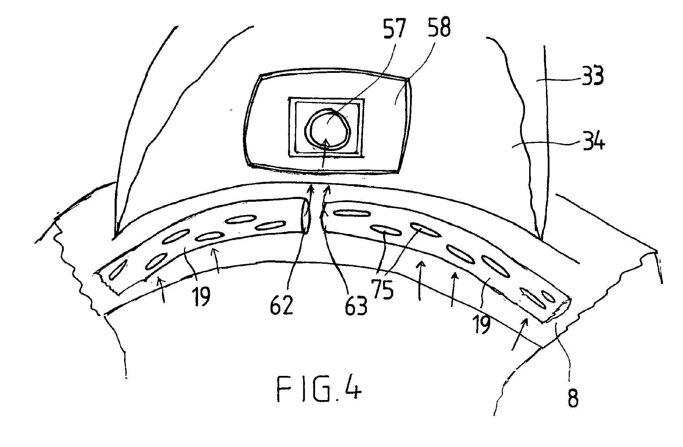

Gemäß, dem in den Fig. 3 und 4 gezeigten Ausführungsbeispiel ist eine gasdichte Abdeckung ausgebildet, die sich aus einer Innenmembran 34 und einer Außenmembran 33 zusammensetzt, die mit ihren Außenrändern an der Beckenkrone 8 gasdicht geklemmt sind. Durch eine druckgeregelte Stützgasatmosphäre wird die Außenmembran 33 immer in einem aufgeblähten Zustand gehalten, während sich die Innenmembran 34 je nach Füllgrad heben oder senken kann. Die Innenmembran 34 schließt das Erdbecken 7 nach oben hin ab und verhindert somit den Austritt des im Erdbeckenfermenter gespeicherten Gases.

Das Rohr 19, welches durch Schlaufen 70 auf dem Betonringfundament 25 fixiert ist, dient dazu, um eine kontrollierte Gasabführung um den gesamten Umfang des Erdbeckens 7 zu ermöglichen. Durch die Durchbrechungen 19 (Fig.4) kann Gas überall entlang des Rohrverlaufs eintreten und im Bereich einer Auslassöffnung 57 austreten (in Fig.4 durch Pfeile verdeutlicht).

In umgekehrter Richtung kann das Rohr 19 auch dazu dienen, über eine Einlassöffnung eintretendes Gas entlang des Erdbeckenumfangs zu verteilen.

Wie in Fig.4 gezeigt ist das Rohr 19 durch eine an einer Stelle offene Ringrohrleitung ausgebildet, deren offene Enden 62, 63 voneinander beabstandet gegenüberliegen und die im Bereich der Gasaustrittsöffnung 57 angeordnet sind, die Teil eines Bedien-Elements 58 ist, welches in der gasdichten Abdeckung, die aus der Innenmembran 34 und der Außenmembran 33 gebildet ist, gasdicht eingesetzt ist.

Die Durchbrechungen in der Rohrwandung sind durch eine Vielzahl von Längsschlitzen 75 gebildet sind, die entlang der Längserstreckung des Rohres 19 verteilt sind.

Die Innenmembran 34 und die Außenmembran 33 sind im Randbereich durch eine sich entlang des gesamten Umfangs der Beckenkrone 8 erstreckenden Klemmschiene 35 auf der Beckenkrone 8 gasdicht geklemmt. Die Klemmschiene 35 ist im gezeigten Ausführungsbeispiel - im Querschnitt gesehen - durch ein Winkelprofil gebildet, das durch im Betonringfundament 25 verankerte Dübelverbindungen 45 fixiert ist.

Innerhalb der gasdichten Abdeckung 33, 34 kann die Erwärmung des Substrats erfolgen, indem auf den Böschungsflächen des abgesenkten Beckenbodens nicht dargstellte Heizungsrohre verlegt sind, welche mit Abstandshalter montiert sein können, um eine Beschädigung der Beckenauskleidung zu vermeiden.

Außerhalb der gasdichten Abdeckung 33, 34 ist eine Vertiefung 28 in der Beckenkrone 8 mit trapezförmigem Querschnitt vorgesehen, die sich entlang des Umfanges der Beckenkrone 8 erstreckt. In der Vertiefung 28 ist ein Drainrohr 29 zur kontrollierten Abführung des Regenwassers angeordnet, wodurch Bodenerosion im Bereich der Beckenkrone 8 verhindert wird.

Um eine möglichst vollständige Drainage zu erreichen, ist die Vertiefung 28 bevorzugt mit Dichtungsbahnen 46 ausgekleidet und mit Kies gefüllt.

Durch entsprechende Abschüssigkeit der Beckenkrone 8 nach außen und nach innen (z.B. 2%) ist garantiert, dass auf der Außenseite Regenwasser in die Vertiefung 8 abrinnt und auf der Innenseite aggressives Kondensat von Innenmembran 34 kontrolliert wieder ins Substrat zurückgeführt wird.

## Patentansprüche

1. Erdbeckenfermenter (1) mit einem abgesenkten Beckenboden (3), der mit einer Dichtungsschicht (31) abgedeckt ist und ein Erdbecken (7) ausbildet, und an dessen Beckenkrone (8) zumindest eine gasdichte Abdeckung (1; 33, 34) festgelegt ist, wobei Zu- und Ableitungen vorgesehen sind, **dadurch gekennzeichnet, dass** innerhalb der gasdichten Abdeckung (1; 33, 34) im Bereich der Beckenkrone (8) zumindest teilweise entlang des Umfangs der Beckenkrone (8) zumindest ein Rohr (19) angeordnet ist, das entlang seiner Längserstreckung Durchbrechungen oder Öffnungen in der Rohrwandung aufweist, durch die hindurch Gas in das Innere des Rohres eintreten oder aus dem Inneren des Rohres austreten kann.

2. Erdbeckenfermenter nach Anspruch 1, **dadurch gekennzeichnet, dass** das zumindest eine Rohr (19) durch eine offene Ringrohrleitung ausgebildet ist, deren offene Enden im Bereich einer Gaseintritts- oder einer Gasaustrittsöffnung angeordnet sind.

3. Erdbeckenfermenter nach Anspruch 1 oder 2, **dadurch gekennzeichnet, dass** die Durchbrechungen in der Rohrwandung durch eine Vielzahl von Längsschlitzen gebildet sind, die entlang der Längserstreckung des Rohres verteilt sind.

4. Erdbeckenfermenter nach Anspruch 1, 2 oder 3, **dadurch gekennzeichnet, dass** in der Beckenkrone (8) ein Betonringfundament (25) eingelassen ist, und dass das Rohr (19) auf dem Betonringfundament (25) angeordnet und gegenüber diesem fixiert ist.

5. Erdbeckenfermenter nach einem der vorhergehenden Ansprüche, **dadurch gekennzeichnet, dass** die gasdichte Abdeckung durch zumindest eine gasdichte Membran (1; 33, 34) gebildet ist.

6. Erdbeckenfermenter nach Anspruch 5, **dadurch gekennzeichnet, dass** die zumindest eine gasdichte Membran durch eine Innenmembran (34) und eine Außenmembran (33) gebildet ist,

7. Erdbeckenfermenter nach einem der vorhergehenden Ansprüche, **dadurch gekennzeichnet, dass** die gasdichte Abdeckung im Randbereich durch eine Klemmschiene (35) auf der Beckenkrone (8) gasdicht geklemmt ist.

8. Erdbeckenfermenter nach Anspruch 7, **dadurch gekennzeichnet, dass** die Klemmschiene (35) durch eine sich entlang des gesamten Umfangs der Beckenkrone (8) erstreckende Klemmschiene (35) gebildet ist.

9. Erdbeckenfermenter nach Anspruch 8, **dadurch gekennzeichnet, dass** die Klemmschiene aus mehreren Einzelschienen zusammengesetzt ist.

10. Erdbeckenfermenter nach Anspruch 7, 8 oder 9, **dadurch gekennzeichnet, dass** die Klemmschiene (35) im Querschnitt gesehen durch ein Winkelprofil gebildet ist.

11. Erdbeckenfermenter nach einem der Ansprüche 7 bis 10, **dadurch gekennzeichnet, dass** die Klemmschiene (35) durch im Betonringfundament verankerte Dübelverbindungen (45) fixiert ist.

12. Erdbeckenfermenter nach einem der vorhergehenden Ansprüche, **dadurch gekennzeichnet, dass** die zumindest eine gasdichte Membran (1; 33, 34) so konfektioniert ist, dass sie im entleerten Zustand des Gasspeichers (7) zumindest teilweise auf dem Speicherboden (3) flächig aufliegt.

13. Erdbeckenfermenter nach Anspruch 12, **dadurch gekennzeichnet, dass** zumindest entlang einem Querschnitt durch den Gasspeicher (7) die Bogenlänge b der zumindest einen Membran (1) im aufgespannten Zustand größer oder gleich der zwischen gegenüberliegenden Einspannpunkten (14) der zumindest einen gasdichten Membran (1) gemessenen Länge der Durchlauflinie d entlang dem Speicherboden (3) ist.

14. Erdbeckenfermenter nach einem der vorhergehenden Ansprüche 1 bis 13, **dadurch gekennzeichnet, dass** außerhalb der gasdichten Abdeckung eine Vertiefung (28) in der Beckenkrone (8) vorgesehen ist, die sich entlang des Umfanges der Beckenkrone (8) erstreckt.

15. Erdbeckenfermenter nach Anspruch 14, **dadurch gekennzeichnet, dass** in der Vertiefung (28) ein Drainrohr (29) zur Abführung des Regenwassers angeordnet ist.

16. Erdbeckenfermenter nach Anspruch 14 oder 15, **dadurch gekennzeichnet, dass** die Vertiefung (28) mit Dichtungsbahnen (46) ausgekleidet und mit Kies gefüllt ist.

17. Erdbeckenfermenter nach einem der vorhergehenden Ansprüche, **dadurch gekennzeichnet, dass** der abgesenkte Beckenboden Böschungsflächen aufweist, auf welchen Heizungsrohre verlegt sind, welche mit Abstandshalter montiert sind.
